# NEUE EUROPÄISCHE PATENTSCHRIFT

(11) **EP 2 058 326 B2**
(45) Veröffentlichungstag und Bekanntmachung des Hinweises auf die Entscheidung über den Einspruch: **15.05.2019**
(45) Hinweis auf die Patenterteilung: 08.07.2015
(21) Anmeldenummer: 09152647.5
(22) Anmeldetag: 14.07.2006
(51) Int. Cl.: C07K 14/435

(54) **Verfahren zur Reinigung von G-CSF**
Method for the purificaiton of G-CSF
Procédé de purification du facteur G-CSF

(30) Priorität: 15.07.2005 DE 102005033250
(43) Veröffentlichungstag der Anmeldung: 13.05.2009
(62) Teilanmeldung aus: 06777781.3
(73) Patentinhaber: Sandoz AG, 4056 Basel (CH)
(72) Erfinder: Dietrich, Arndt, 08468 Reichenbach (DE); Janowski, Bernhard, 06118 Halle (DE); Schäffner, Jörg, 06198 Salzmünde (DE); Blaschke, Ulrich, 65189 Wiesbaden (DE)
(74) Vertreter: Elkington and Fife LLP

(56) Entgegenhaltungen:
- EP-A2- 0 344 796
- WO-A-01/04154
- WO-A-03/051922
- WO-A-2005/049062
- WO-A1-01/04329
- US-A1- 2002 004 483
- NOMURA H ET AL: "PURIFICATION AND CHARACTERIZATION OF HUMAN GRANULOCYTE COLONY-STIMULATING FACTOR (G-CSF)" EMBO JOURNAL, IRL PRESS, EYNSHAM, GB, Bd. 5, Nr. 5, 1. Mai 1986 (1986-05-01), Seiten 871-876, XP000650289 ISSN: 0261-4189
- ISHIZAKA Y ET AL: "MODE OF ACTION OF HUMAN URINARY COLONY-STIMULATING FACTOR" BIOSIS, 1986, XP002133881
- NICOLA N A ET AL: "PURIFICATION OF A FACTOR INDUCING DIFFERENTIATION IN MURINE MYELO MONOCYTIC LEUKEMIA CELLS IDENTIFICATION AS GRANULOCYTE COLONY STIMULATING FACTOR" BIOSIS, 1984, XP002133879
- Englische Übersetzung der Korianischen Patentanmeldung 1996-000649
- Garg V.K. et al: "Purificatioin and Production of Therapeutic Grade Proteins", Bioprocess Technology, vol. 4, 1991, pages 29-54, New York
- Dietrich et al: "Industrial Protein folding, BIOforum Europe", January 2003 (2003-01), Git Verlag, Darmstadt pages 34-36,
- Pailhes et al: BioProcessing Journal, vol. 3, no. 3, April 2004 (2004-04), - May 2004 (2004-05), pages 55-58,
- Todd E. Arnold: Bioprocess International, November 2005 (2005-11), pages 44-49,
- "Protein Concentration and Diafiltration by tangential flow fitration", Millipore, 2003

## Beschreibung

Die Erfindung betrifft ein Verfahren zur Reinigung von G-CSF aus einem Gemisch von G-CSF und anderen Proteinen, umfassend zwei Kationenaustauschchromatographieschritte, die jeweils vor und nach einer hydrophoben Interaktionschromatographie durchgeführt werden, wobei die chromatographiesschritte unmittlebar aufeinander folgen. G-CSF (granulocyte colony stimulating factor) ist ein natürlich vorkommender Wachstumsfaktor, der zur Familie der Cytokine im weiteren Sinne und hier zur Gruppe der Koloniestimulierenden Faktoren gehört. G-CSF spielt eine entscheidende Rolle bei der Hämatopoese und fordert die Proliferation und Differenzierung von hämatopoetischen Vorläuferzellen und die Aktivierung von Neutrophilen. Aufgrund dieser Eigenschaften hat G-CSF Anwendung in verschiedenen medizinischen Bereichen gefunden, wie z.B. der Rekonstitution normaler Blutzellpopulationen nach der Chemotherapie oder Bestrahlung, oder zur Stimulation der Immunantwort gegenüber infektiösen Pathogenen. So wird G-CSF in der Klinik hauptsächlich bei der Tumorbekämpfung und insbesondere zur Behandlung von Neutropenie als Folge von Chemotherapie eingesetzt und findet ferner auch bei Knochenmarkstransplantationen und bei der Behandlung von Infektionskrankheiten Verwendung.

Humanes G-CSF in seiner natürlich vorkommenden Form ist ein Glycoprotein mit einem Molekulargewicht von etwa 20.000 Dalton und weist fünf Cystein-Reste auf. Vier dieser Reste bilden zwei intramolekulare Disulfidbrücken, die für die Aktivität des Proteins von wesentlicher Bedeutung sind. Da G-CSF aus seinen natürlichen Quellen nur in geringen Mengen erhältlich ist, werden zur Herstellung von Arzneimitteln hauptsächlich rekombinante Formen von G-CSF verwendet, die beispielsweise durch Expression in Säugerzellen wie CHO (Chinese Hamster Ovary)-Zellen oder Prokaryontenzellen wie *E. coli* erhalten werden können. Die in Säugerzellen exprimierten rekombinanten Proteine unterscheiden sich von natürlich vorkommendem G-CSF durch ein unterschiedliches Glykosylierungsmuster, während bei den in *E. coli* exprimierten Proteinen, die als Folge der bakteriellen Expression einen zusätzlichen N-terminalen Methioninrest aufweisen können, die Glykosylierung vollständig fehlt.

Die rekombinante Herstellung von G-CSF wurde in der Patentliteratur erstmals 1987 beschrieben, in WO 87/01132 A1. Das erste kommerzielle G-CSF-Präparat auf der Basis von rekombinantem G-CSF wurde in Deutschland 1991 zugelassen und wird unter dem Handelsnamen Neupogen^{®} von der Firma Amgen hergestellt und vertrieben.

Zwar ist die Produktion von G-CSF in prokaryotischen Zellen gegenüber der Produktion in Säugerzellen bevorzugt, da einfachere Expressionssysteme und Kulturbedingungen verwendet werden können, doch ist ein häufig auftretenden Problem bei der Herstellung von rekombinanten Proteinen in prokaryotischen Zellen die Bildung von schwerlöslichen intrazellulären Aggregaten von denaturierten Formen des exprimierten Proteins, den sog. Inclusion Bodies, die teilweise eine Sekundärstruktur aufweisen und im Zytoplasma der Bakterienzellen aufgefunden werden können.

Die Bildung dieser Inclusion Bodies führt dazu, dass die Proteine nach der Isolierung der Inclusion Bodies durch Zentrifugation bei mäßiger Geschwindigkeit durch geeignete Mittel solubilisert und renaturiert werden müssen, um ihre aktive Konfiguration zu erhalten. Dabei ist die Konkurrenzreaktion zwischen einer Überführung des denaturierten Proteins in das richtige Faltungsintermediat und einer Aggregation von mehreren Proteinmolekülen ein wesentlicher, die Ausbeute an renaturiertem Protein limitierender Faktor.

Im Stand der Technik beschäftigen sich diverse Patentdokumente mit dem Aspekt der Solubilisierung und Renaturierung von in Inclusion Bodies gewonnenen Proteinen. In EP-A-0 719 860 beispielsweise werden die Isolierung und Aufreinigung von G-CSF, einschließlich der Solubilisierung und Rückfaltung beschrieben. Allgemeine Techniken zur Solubilisierung und Renaturierung von denaturierten Proteinen sind in EP-A-0 512 097, EP-A-0 364 926, EP-A-0 219 874 und WO 01/87925 beschrieben worden und können außerdem der wissenschaftlichen Literatur und Standardwerken der Proteinchemie entnommen werden.

Das rückgefaltete Protein wird anschließend mittels chromatographischer Methoden gereinigt, d.h. es wird von anderen Proteinen und sonstigen Verunreinigungen, die nach der Solubilisierung und Renaturierung enthalten sind, getrennt.

Mit der chromatographischen Aufreinigung beschäftigt sich auch die bereits genannte WO 87/01132 A1, in der erstmals die Herstellung von G-CSF in *E. coli-*Wirtszellen beschrieben wurde. Im Rahmen der Reinigung des rekombinanten G-CSF wird in WO 87/01132 A1 in Beispiel 7 eine Kationenaustauschchromatographie unter Verwendung einer CM-Cellulose-Säule durchgeführt.

In EP 0 719 860 A1 wird das G-CSF im Anschluss an die Solubilisierung und Oxidation mittels Dowex zur Entfernung des Solubilisierungsmittels, gefolgt von einer Anionenaustauschchromatographie und einer Kationenaustauschchromatographie gereinigt. Auch in EP 0 719 860 A1 wird für die Kationenaustauschchromatographie CM-Sepharose verwendet.

In WO 03/051922 A1 wird ein Reinigungsverfahren für G-CSF beschrieben, bei dem eine Metallaffinitätschromatographie durchgeführt wird, genauer gesagt, eine Chromatographie an immobilisiertem Metall (immobilized metal affinity chromatography, IMAC). Im Anschluss an die Metallaffinitätschromatographie kann in WO 03/051922 eine Kationenaustauschchromatographie und/oder eine Gelfiltration stattfinden.

In WO 01/04154 A1 ist ein Verfahren zur Reinigung von G-CSF beschrieben, bei dem zunächst eine hydrophobe Interaktionschromatographie und darauf folgend eine Hydroxyapatit-Chromatographie durchgeführt werden. Im Anschluss an die Hydroxyapatit-Chromatographie erfolgt eine Kationenaustauschchromatographie.

Aufgabe der vorliegenden Erfindung ist es, ein Verfahren zur Reinigung von biologisch aktivem rekombinanten humanen G-CSF aufzuzeigen, mit dem G-CSF in zufriedenstellender Reinheit und Ausbeute gewonnen werden kann. Dabei soll das Verfahren möglichst einfach und überschaubar in der Durchführung sein. Wünschenswert ist ein Reinigungsverfahren, das mit möglichst wenigen chromatographischen Schritten auskommt, um den technischen Aufwand und die Kosten gering zu halten und hohe Proteinverluste zu vermeiden.

Diese und weitere Aufgaben werden durch das in Anspruch 1 angegebene Verfahren gelöst. Bevorzugte Ausführungsformen sind in den abhängigen Patentansprüchen beschrieben.

Es wurde gefunden, dass bei chromatographischer Reinigung von renaturiertem G-CSF mittels einer Kationenaustauschchromatographie und einer hydrophoben Interaktionschromatographie eine akzeptable Reinheit des rekombinanten, biologisch aktiven G-CSF bei zufriedenstellender Ausbeute erzielt werden kann. Die Reinheit wird durch einen zweiten Kationenaustauschchromatographieschritt weiter gesteigert.

Es wird somit ein Verfahren zur Reinigung von rekombinant hergestelltem biologisch aktiven humanen G-CSF beschrieben, bei dem zwei Kationenaustauschchromatographie schritte und eine hydrophobe Interaktionschromatographie durchgeführt werden, wobei diese Chromatographieschritte in beliebiger Reihenfolge stattfinden, allerdings ohne dass zwischen diesen Schritten ein anderer Chromatographieschritt oder anderer Reinigungsschritt erfolgt. Die Kationenaustauschchromatographie und die hydrophobe Interaktionschromatographie folgen also unmittelbar aufeinander.

Unter "biologisch aktivem humanen G-CSF" wird erfindungsgemäß verstanden, dass das durch das erfindungsgemäße Verfahren gereinigte G-CSF in der Lage ist, die Differenzierung und Proliferation von hämatopoetischen Vorläuferzellen zu fördem und die Aktivierung von reifen Zellen des hämatopoetischen Systems zu bewirken.

Daher eignet sich das G-CSF, das durch das erfindungsgemäße Verfahren gewonnen wurde, zur Behandlung von Indikationen, bei denen die Verabreichung von G-CSF vorteilhaft ist. Es versteht sich, dass der Begriff "biologisch aktives humanes G-CSF" auch Mutanten und Modifikationen von G-CSF einschließt, deren Aminosäuresequenz gegenüber der Wildtypsequenz verändert ist, die aber eine ähnliche biologische Aktivität wie das Wildtyp-G-CSF aufweisen, wie z. B. die in WO 01/87925 und EP 0 456 200 beschriebenen. Gleiches gilt für G-CSF-Konjugate. Bevorzugt handelt es sich bei dem zu reinigenden G-CSF um humanes Met-G-CSF, hergestellt in E. coli-Zellen.

In einer weiteren Ausführungsform der Erfindung umfasst das Verfahren eine Tangentialflussfiltration im Anschluss an die letzte Kationenaustauschchromatographie.

In einer weiteren Ausführungsform wird in dem Reinigungsverfahren für G-CSF auf die Durchführung einer Anionenaustauschchromatographie verzichtet.

In einer weiteren Ausführungsform kommt das erfindungsgemäße Reinigungsverfahren ohne Gelfiltrationschromatographie aus.

In einer weiteren Ausführungsform der Erfindung wird auf die Durchführung einer präparativen HPLC verzichtet. Gleiches gilt für reversed phase Chromatographie, die von der erfindungsgemäßen hydrophoben Interaktionschromatographie zu unterscheiden ist und auf die ebenfalls in der Präparation möglichst verzichtet wird. rpHPLC wird lediglich zu analytischen Zwecken eingesetzt.

In einer weiteren Ausführungsform findet im Rahmen des Verfahrens keine Affinitätschromatographie, insbesondere keine Farbstoff-, Metall- oder Immunglobulin-Affinitätschromatographie statt.

In einer weiteren Ausführungsfonn wird im Rahmen des Reinigungsverfahrens auf die Durchführung einer Hydroxyapatitchromatographie verzichtet.

Das erfindungsgemäße Reinigungsverfahren macht somit in einer bevorzugten Ausführungsform von lediglich zwei verschiedenen chromatographischen Trennmethoden Gebrauch, nämlich der des Ionenaustausches auf der Grundlage der kompetitiven Wechselwirkung geladener Ionen und der der hydrophoben Interaktion, die sich dadurch auszeichnet, dass die unpolaren Oberflächenregionen eines Proteins bei hohen Salzkonzentrationen an die schwach hydrophoben Liganden einer stationären Phase adsorbieren.

Hiervon zu unterscheiden ist das chromatographische Trennprinzip der Affinität, die auf der spezifischen und reversiblen Adsorption eines Moleküls an einen individuellen, matrixgebundenen Bindungspartner beruht. Bei der Hydroxyapatit-Chromatographie, die auf dem Einsatz von anorganischen Hydroxyapatitkristallen basiert, handelt es sich um ein weitere Trennmethode, die sich von der Ionenaustauschchromatographie in Form der Kationenaustauschchromatographie und der hydrophoben Interaktionschromatographie unterscheidet.

Diese genannten Chromatographieprinzipien werden auch in Fachkreisen entsprechend unterschieden (siehe z.B. Bioanalytik, F. Lottspeich, H. Zorbas (Hrsg.), Heidelberg, Berlin, Spektrum Akad. Verlag 1998).

In einer bevorzugten Ausführungsform umfasst die chromatographische Reinigung nicht mehr als drei Chromatographieschritte, in denen nur zwei verschiedene chromatographische Trennmethoden zum Einsatz kommen.

Als Ausgangsmaterial für die chromatographische Reinigung wird renaturiertes G-CSF eingesetzt, das in eine Reinheit überführt werden soll, die seine Verwendung in Form einer pharmazeutischen Zubereitung ermöglicht.

Die Solubilisierung und Rückfaltung des Proteins kann dabei nach den in der Technik bekannten Methoden erfolgen, bspw. wie in EP-A-1 630 173 beschrieben.

Das rückgefaltete G-CSF kann nach der Rückfaltung und vor dem ersten Chromatographieschritt aufbereitet werden, z.B. durch Filtration, Konzentrierung, Fällung, Ansäuerung und/oder Dialyse.

In vielen Fällen wird es vorteilhaft sein, den Faltungsansatz vor dem ersten Chromatographieschritt zu klären, d.h. höhermolekulare Partikel zu entfernen, bei denen es sich vor allem um Proteinaggregate handelt, die bei der Faltung gebildet wurden. Diese Klärung kann durch Tiefenfiltration erfolgen, bei der eine lockere Schüttung eines körnigen Materials als Filtermittel dient. Die Feststoffteilchen sind größer als die Poren des Filtermittels oder werden durch Absorption an der inneren Oberfläche der Schüttung zurückgehalten.

Bevorzugt werden für die Tiefenfiltration Celluloseester-Fasern als Filtermittel eingesetzt. Geeignete Filtermaterialien sowie entsprechende Anwendungsvorschriften sind z.B. erhältlich von der Firma Millipore unter den Handelsnamen Millistak Plus C0HC und Millistak + B1HC.

Bevorzugt wird vor der Tiefenfiltration der Faltungsansatz angesäuert, so dass das Filtrat besonders effizient unmittelbar für die Kationenaustauschchromatographie eingesetzt werden kann. Bevorzugt wird der pH-Wert des Faltungsansatzes hierbei auf unter 4,0, besonders bevorzugt auf 3,2 eingestellt.

Für die Kationenaustauschchromatographie können übliche, kommerziell erhältliche Matrices eingesetzt werden. Dabei bindet das G-CSF in einem bestimmten pH-Bereich aufgrund seiner positiven Gesamtladung an die Kationenaustauschmatrix, während die meisten kontaminierenden Stoffe, wie Nukleinsäuren, Lipopolysaccharide und Proteine, die aus den Wirtszellen stammen, sowie ionische Isomere von G-CSF und veränderte Formen von G-CSF mit anderen pI-Werten nicht binden oder durch Waschen entfernt werden können.

Geeignete Kationaustauschmatrices schließen ein, sind aber nicht beschränkt auf, Carboxymethyl (CM)-Cellulose, AG 50 W, Bio-Rex 70, Carboxymethyl (CM)-Sephadex, Sulfopropyl (SP)-Sephadex, Carboxymethyl (CM)-Sepharose CL-6B, CM-Sepharose HP, Hyper D-S-Ceramic (Biosepra) und Sulfonat (S)-Sepharose, SP Sepharose FF, SP Sepharose HP, SP Sepharose XL, CM Sepharose FF, TSK gel SP 5PW, TSK gel SP-5PW-HR, Toyopearl SP-650M, Toyopearl SP-650S, Toyopearl SP-650C, Toyopearl CM-650M, Toyopearl CM-650S, Macro-Prep High S Support, Macro-Prep S Support, Macro-Prep CM Support etc.

Geeignete Matrices und Protokolle zur Durchführung der Kationenaustauschchromatographie kann der Fachmann den Produktinformationen von Anbietern wie Amersham Biosciences (http://www.amershambiosciences.com, inzwischen GE Healthcare) oder Bio-Rad (http://www.bio-rad.com) entnehmen.

Bevorzugt werden Sulfopropylmatrices, insbesondere die Produkte SP Sepharose XL und SP-Sepharose FF (Fast Flow), erhältlich von Amersham Biosciences, Freiburg, Deutschland (inzwischen GE Healthcare), als Matrix für die Kationenaustauschchromatographie verwendet.

In einer bevorzugten Ausführungsform der Erfindung, wird in den zwei Kationenaustauschchromatogra- phien in beiden Fällen eine Sulfopropylmatrix eingesetzt, be- sonders bevorzugt in der ersten Kationenaustauschchromatographie SP Sepharose XL und in der zweiten Kationenaustauschchromatographie SP Sepharose FF.

Geeignete Puffer für die Kationenaustauschchromatographie schließen ein Maleat-, Malonat-, Citrat-, Lactat-, Acetat-, Phosphat-, HEPES- und Bicin-Puffer. Die Konzentration des Puffers liegt bevorzugt zwischen 10 und 100 mM, bevorzugt zwischen 20 mM und 50 mM. Der pH des Puffers sollte für die Aufreinigung des G-CSF möglichst nicht höher als 7,0, bevorzugt nicht höher als 6,5 sein.

In einer bevorzugten Ausführungsform wird für die Kationenaustauschchromatographie 20 mM Natriumacetat pH 5,0 verwendet, das zum Equilibrieren und Waschen eingesetzt wird.

In der zweiten Kationenaustauschchromatographie, wird hierbei bevorzugt 50 mM Natriumphosphat, pH 5,4 zum Equilibrieren und Waschen verwendet.

Das G-CSF kann nach dem Waschen durch eine Veränderung, im Falle der Kationenaustauschchromatographie durch eine Erhöhung, des pH-Wertes oder eine Erhöhung der Ionenstärke von der Säule eluiert werden.

Bevorzugt wird die Elution durch Erhöhung der Ionenstärke bewirkt. Wenn 20 mM Natriumacetat pH 5,0 als Puffer verwendet wird, eignet sich für die Elution beispielsweise ein Lösung von 20 mM Natriumacetat pH 5,0 und 200 mM NaCl.

Weitere geeignete Bedingungen für die Kationenaustauschchromatographie können der einschlägigen Literatur, wie etwa dem Handbuch "Ion Exchange Chromatography - Principles and Methods" von Amersham Biosciences, Freiburg, Deutschland (inzwischen GE Healthcare), 2002, entnommen werden.

Die Salzkonzentration im Ladungspuffer für die Kationenaustauschchromatographie sollte ausreichend niedrig sein, um das Binden an die Matrix zu ermöglichen, wobei die Bindung auch von dem pH-Wert der Lösung abhängt.

Verschiedene Puffer können für das Laden und das Binden an die Matrix im Rahmen der Kationenaustauschchromatographie eingesetzt werden, z. B. Puffer ausgewählt aus der Gruppe bestehend aus Acetat, Citrat, Tris/HCl, Tris/Acetat, Phosphat, Succinat, Malonat, 2-(N-Morpholinoethansulfonat) (MES) und andere Puffer.

Nach dem Laden der Säule wird die Säule gewaschen und anschließend werden die Proteine von der Säule eluiert. Die Elution kann dabei durch Erhöhen der Ionenstärke, was durch Erhöhen der Salzkonzentration in der Pufferlösung bewirkt wird, erfolgen. Alternativ bietet sich eine Erhöhung des pH-Werts an. Dabei können diskontinuierliche Stufengradienten, lineare Gradienten oder eine geeignete Kombination derartiger Gradienten verwendet werden.

Elutionspuffer, die für das Waschen und die Elution geeignet sind, können ausgewählt werden aus Acetat, Citrat, Tris/HCl, Tris/Acetat, Phosphat, Succinat, Malonat, MES und anderen geeigneten Puffern mit Zugabe von Salzen wie NaCl oder KCl. Die Ionenstärke und die Salzkonzentration, durch welche die Elution erreicht wird, ist vom pH-Wert der Pufferlösung abhängig. Je höher der pH-Wert des Puffers ist, desto niedriger ist die für die Elution der Proteine von der Säule erforderliche Ionenstärke.

Auch die hydrophobe Interaktionschromatographie kann mit üblichen Matrices durchgeführt werden. Geeignet sind Matrices wie Butyl-, Phenyl- oder Octyl-Sepharose (Amersham Biosciences, inzwischen GE Healthcare), Makro-Prep-Methyl oder t-Butyl (Bio-Rad) und Fractogel EMD mit Propyl- oder Phenyl-Liganden (Merck).

Bevorzugt handelt es sich bei den hydrophoben Liganden um Butyl-, Phenyl- oder Octylgruppen, besonders bevorzugt um Phenylgruppen. Dabei können die Produkte von Amersham Biosciences (inzwischen GE Healthcare) verwendet werden.

Geeignete Matrices und Protokolle zur Durchführung der hydrophoben Interaktionschromatographie kann der Fachmann den Produktinformationen von Anbietern wie Amersham Biosciences (http://www.amershambiosciences.com, inzwischen GE Healthcare) oder Bio-Rad (http://www.bio-rad.com) entnehmen.

Bevorzugt handelt es sich bei der Matrix um Phenyl Sepharose HP (High Performance), erhältlich von Amersham Biosciences (inzwischen GE Healthcare).

Als Puffer für die hydrophobe Interaktionschromatographie eignen sich übliche auch in anderen Chromatographiearten eingesetzte Puffer. In einer bevorzugten Ausführungsform wird ein Citratpuffer eingesetzt. Die Elution erfolgt vorteilhafterweise durch Erhöhung des pH-Wertes. Als besonders geeignet hat sich ein pH-Gradient von etwa pH 3,0 auf ca. 6,0 erwiesen.

Weitere geeignete Bedingungen für die hydrophobe Interaktionschromatographie können der einschlägigen Literatur, wie etwa dem Handbuch "Hydrophobic Interaction Chromatographie - Principles and Methods" von Amersham Biosciences, Freiburg, Deutschland, 2002, entnommen werden.

Allgemein sind die in dem erfindungsgemäßen Verfahren genutzten chromatographischen Prinzipien dem Fachmann geläufig, jedenfalls in gängigen Manuals oder Protokollen der Anbieter von Chromatographiematrices, -säulen und anderen Hilfsmitteln ausführlich beschrieben.

Die im Rahmen einer Ausführungsform der Erfindung im Anschluss an die chromatographische Reinigung, insbesondere im Anschluss an die letzte Kationenaustauschchromatographie durchgeführte Tangential Flow-Filtration (TFF) kann mit herkömmlichen TFF-Systemen und -Protokollen erfolgen, wie sie beispielsweise von den Firmen Millipore und Pall Corporation angeboten werden. Bei der TFF handelt es sich um eine Filtration als zusätzlichen Reinigungsschritt im Unterschied zu den vorangegangenen Reinigungsschritten der Kationenaustausch- und hydrophoben Interaktionschromatographie.

Das im Rahmen der Erfindung gereinigte G-CSF wird mittels herkömmlicher gentechnologischer Methoden in Wirtszellen exprimiert. Bevorzugt handelt es sich dabei um humanes G-CSF. Für die Expression in *E. coli*-Zellen sind verschiedene Expressionssysteme kommerziell erhältlich. Geeignet ist beispielsweise die Expression von humanem G-CSF unter den Kontrolle eines induzierbaren Promotors, beispielsweise eines IPTG-induzierbaren Promotors, siehe z. B. Sambrook and Russel, Molecular Cloning - A Laboratory Manual, 3. Auflage 2001, Coldspring Harbour Laboratory Press, Coldspring Harbour, NY, Kapitel 15, oder gängige Herstellerprotokolle, z. B. von Promega oder Stratagene.

Die Fermentation erfolgt nach Standardprotokollen, wie sie in der Patent- und wissenschaftlichen Literatur beschrieben sind, beispielsweise in einem zweistufigen Prozess, bestehend aus einer Batch-Kultivierung und einer Fed-Batch-Kultivierung.

Die Ernte der so genannten Inclusion Bodies, die das in *E. coli* überexprimierte G-CSF enthalten, und der Aufschluss dieser Einschlusskörper sind teilweise in der oben diskutierten Patentliteratur beschrieben. Geeignete Protokolle finden sich aber auch in Standardwerken der Proteinchemie sowie in Laborhandbüchern. Gleiches gilt für die Solubilisierung und die Rückfaltung, die, wie oben diskutiert, Gegenstand diverser Patentschriften sind.

Bereitgestellt werden auch pharmazeutische Zubereitungen, die das erfindungsgemäß gewonnene G-CSF enthalten. Das gewonnene G-CSF kann entweder als Lyophilisat oder in flüssiger Form aufbewahrt werden. Es wird entweder subkutan oder intravenös verabreicht. Geeignete Hilfsstoffe in den Formulierungen des rekombinant exprimierten G-CSF sind etwa Stabilisatoren wie Zucker und Zuckeralkohole, Aminosäuren sowie Tenside wie z.B. Polysorbat 20/80 sowie geeignete Puffersubstanzen. Beispiele für Formulierungen sind beschrieben in EP 0 674 525, EP 0 373 679 sowie EP 0 306 824, siehe auch Handelsprodukte Neupogen^{®} und Granocyte in ROTE LISTE 2004.

### Beispiele

Die folgenden Beispiele sollen die Erfindung erläutern, ohne sie einzuschränken.

Das humane G-CSF wurde unter der Kontrolle eines IPTG-induzierbaren Promotors in *E. coli*-Zellen exprimiert. Beispiele für geeignete Expressionssysteme können z.B. dem Laborhandbuch Sambrook und Russell, Molecular Cloning - A Laboratory Manual, 3. Auflage 2001, Cold Spring Harbor Laboratory Press, Cold Spring Harbor, NY, Kapitel 15, oder gängigen Herstellerprotokollen, z.B. von Promega oder Stratagene, entnommen werden.

Die Fermentation erfolgte nach Standardprotokollen, wie sie in der Patent- und wissenschaftlichen Literatur beschrieben sind, in einem zweistufigen Prozess umfassend eine Batch-Kultivierung und eine Fed-Batch-Kultivierung. Die Bakterien wurden für 17 bis 18 Stunden kultiviert, bevor sie durch Zugabe von 1 mM IPTG zur Bildung des rekombinanten G-CSF angeregt wurden. Die Induktionsdauer betrug 4,0 Stunden.

Die Ernte der Bakterien erfolgte durch Becherzentrifugation für 20 Minuten bei 5000 g und 4°C. Nach der Zentrifugation wurde der Überstand verworfen und die Zellen mit Puffer (20 mM Natriumphosphat, pH 7,0; 1 mM EDTA) wieder auf das Fermentationsvolumen aufgefüllt, bevor sie durch drei Passagen bei 800 bar aufgeschlossen wurden. Anschließend wurde das Lysat durch Separation (CSA1-Separator, Westfalia, Oelde) geklärt.

### Konzentrieren der Inclusion Bodies

Prinzipiell kann die durch die Ernte gewonnene Suspension der Inclusion Bodies direkt für die nachfolgende Solubilisierung eingesetzt werden. Allerdings ist in diesem Fall die maximal erreichbare Proteinkonzentration im Solubilisat stark limitiert, was zur Einschränkung während der Faltung führen kann. Daher sollte die Suspension der Inclusion Bodies nach der Ernte und dem Waschen durch Zentrifugation konzentriert werden, um eine hohe Proteinkonzentration im Solubilisat zu erreichen.

Die Suspension der Inclusion Bodies wurde für 20 Minuten bei 10.000 g in einer Becherzentrifuge zentrifugiert. Die durch die Zentrifugation gewonnene Paste der Inclusion Bodies kann für mindestens 12 Wochen bei -20°C gelagert werden.

### Solubilisierung

Um größere Pellets von Inclusion Bodies in einer relativ kurzen Zeit effektiv solubilisieren zu können, ist außerdem eine mechanische Zerkleinerung dieser Pellets, etwa durch Ultraturrax-Behandlung, notwendig. Die durch Zentrifugation erhaltene Paste der Inclusion Bodies wurde gewogen, mit 9,0 ml Solubilisierungspuffer (30 mM Tris, 1 mM EDTA, 6,0 M Guanidin-HCl, 100 mM GSH, pH 8,0) pro Gramm Inclusion Bodies versetzt und durch Ultraturrax-Behandlung zerkleinert. Der Ansatz wurde gründlich gevortext und dann auf einem Roller-Mixer oder einem Magnetrührer bei Raumtemperatur für etwa 2 Stunden inkubiert.

### Rückfaltung

Die Proteinkonzentration im Solubilisat wurde mit der Methode nach Bradford mit BSA als Standardprotein bestimmt. Für die Faltung wurde die Menge Solubilisat zum Rückfaltungspuffer (30 mM Tris, 2 mM GSSG, 2 mM GSH, 3 M Harnstoff, pH 7,5; 4°C) zugegeben, die benötigt wurde, um in einer gewünschten Menge Puffer eine Proteinkonzentration von 700 µg/ml zu erzielen. Die entsprechende Menge an Solubilisat wurde unter Rühren mit einem Magnetrührer langsam und gleichmäßig zugegeben, um lokal erhöhte Konzentrationen an Solubilisat bzw. Protein zu vermeiden. Die Zuflussgeschwindigkeit und die Art der Durchmischung kann für das jeweilig verwendete Volumen des Solubilisierungsansatzes angepasst werden. Nach abgeschlossener Zugabe des Solubilisates wurde der Ansatz für mindestens 12 Stunden bei 4°C inkubiert. Während dieser Zeit war ein weiteres Mischen nicht erforderlich.

### Tiefenfiltration

Im Anschluss an die Rückfaltung wird der Rückfaltungsansatz filtriert, bevor der erste Chromatographieschritt stattfindet. Dabei kann für die Filtration beispielsweise ein Tiefenfilter eingesetzt werden, z.B. ein geeigneter Filter von der Firma Millipore, Schwalbach. Vor der Filtration wird der pH-Wert mit 2 M Citronensäure auf pH 3,2 eingestellt.

### Durchführung der ersten Kationenaustauschchromatographie

Der erste Chromatographieschritt dient dem Capture des Zielproteins und trennt Rückfaltungshilfsmittel wie Harnstoff, GSH, GSSG, soweit im Faltungsansatz vorhanden, vom Zielprotein. In diesem Schritt werden auch falsch gefaltete Proteinspezies und Wirtszellproteine abgetrennt. Hier kommt eine Kationenaustauschchromatographie zum Einsatz. Als Matrix wird SP Sepharose XL von Amersham Biosciences (inzwischen GE Healthcare) verwendet. Die Chromatographie findet bei pH 5,0 statt.

Die SP Sepharose XL-Matrix wurde mit 1,5 Säulenvolumen von 20 mM Natriumacetat, pH 5,0 equilibriert. Der filtrierte Rückfaltungsansatz wurde auf die Säule geladen und anschließend mit 1,5 Säulenvolumen Waschpuffer (20 mM Natriumacetat, pH 5,0) gewaschen. Anschließend wurde mit 3 Säulenvolumen Elutionspuffer (20 mM Natriumacetat, 200 mM NaCl, pH 5,0) das G-CSF von der Säule eluiert. Die Reinheit des eluierten G-CSF wurde mittels rpHPLC bestimmt, sie betrug über 80%. Die Ausbeute lag, bezogen auf den filtrierten Faltungsansatz, ebenfalls bei über 80%

### Durchführung der hydrophoben Interaktionschromatographie

Im zweiten Chromatographieschritt findet, ausgehend vom Eluat der SP Sepharose XL, eine weitere Reinigung des G-CSF statt. Insbesondere die produktverwandten Verunreinigungen werden wesentlich abgereichert. Hier kommt eine hydrophobe Interaktionschromatographie mit Phenyl Sepharose HP von Amersham Biosciences (inzwischen GE Healthcare) zum Einsatz.

Die Phenyl Sepharose HP-Säule wurde zunächst mit 2 Säulenvolumen 12% Puffer B, 88% Puffer A (Puffer B: 20 mM Natriumcitrat, pH 6,7; Puffer A: 20 mM Natriumcitrat, pH 2,7, 110 mM NaCl) equilibriert. Dann wurde das Eluat der SP Sepharose XL-Säule, das zuvor mit 5 Volumen von Puffer A (20 mM Natriumcitrat, pH 2,7, 110 mM NaCl) verdünnt worden war, auf die Säule aufgetragen. Anschließend wurde die Säule mit 2 Säulenvolumen 12% Puffer B, 88% Puffer A gewaschen und ein linearer Gradient von 12% auf 90% Puffer B in 5-8 Säulenvolumen gefahren. Im Rahmen dieses linearen pH-Gradienten von etwa pH 3,0 auf ca. 6,0 fand die Elution statt. Schließlich wurde die Säule mit 3 Säulenvolumina 90% Puffer B, 10% Puffer A gespült.

Die Elutionsfraktionen wurden mittels rpHPLC auf ihre Reinheit überprüft und Fraktionen mit über 95% Reinheit zusammengefasst.

Das nach der hydrophoben Interaktionschromatographie erhaltene G-CSF hatte eine Reinheit von über 96 %. Die Ausbeute aus dem HIC-Schritt entsprach fast 80%.

### Durchführung der zweiten Kationenaustauschchromatographie

Im dritten Chromatographieschritt findet, ausgehend vom Eluat der Phenyl Sepharose HP, eine weitere Reinigung des G-CSF bis zu einer Reinheit von über 99% statt. Insbesondere die produktverwandten Verunreinigungen werden wesentlich abgereichert. Hier kommt wiederum eine Kationenaustauschchromatographie zum Einsatz. Dabei wird SP Sepharose FF von Amersham Biosciences (inzwischen GE Healthcare) verwendet.

Die SP Sepharose FF-Säule wurde mit 3 Säulenvolumen 100% Puffer A (50 mM Natriumphosphat, pH 5,4) equilibriert. Anschießend wurde das Eluat der hydrophoben Interaktionschromatographie auf die Säule aufgetragen und die Säule mit 2 Säulenvolumen 100% Puffer A (50 mM Natriumphosphat, pH 5,4) gespült. Die Auftragsprobe enthielt dabei ca. 60 mM NaCl und hatte einen pH-Wert von 4,0-4,2. Die Elution erfolgte mittels einer Kombination aus Stufen- und linearem pH-Gradient. Die erste Stufe ging auf 10% Puffer B (50 mM Natriumphosphat, pH 6,4) und hielt diese Konzentration für 1,5 Säulenvolumen. Danach folgte ein Gradient über 1 Säulenvolumen von 10 auf 15% Puffer B (50 mM Natriumphosphat, pH 6,4). G-CSF wurde in einem linearen Gradienten von 15% auf 35% Puffer B (50 mM Natriumphosphat, pH 6,4) über 12,5 Säulenvolumen eluiert, wobei das Sammeln des Eluats mit Anstieg der Absorption bei 280 mm erfolgte. Schließlich wurde die Säule mit einer Stufe auf 100% Puffer B (50 mM Natriumphosphat, pH 6,4) gespült.

Die Elutionsfraktionen wurden mittels rpHPLC auf ihre Reinheit überprüft und Fraktionen mit über 99% Reinheit zusammengefasst. Die Gesamtausbeute lag bei 80%.

Das als Ergebnis der oben beschriebenen Chromatographieschritte erhaltene Met-G-CSF hatte eine Reinheit von mindestens 99,5% nach allen HPLC-Analysen (rp, SEC und IEX).

Die Bestimmung der Verunreinigungen ergab ebenfalls eine sehr starke Abreicherung von DNA, Endotoxin und Wirtszellprotein.

### Bestimmung der biologischen Aktivität

Die Aktivität des durch das erfindungsgemäße Verfahren gewonnenen G-CSF wurde durch einen Bioassay bestimmt und mit der eines Standards, kommerziell erhältlichem G-CSF (Neupogen^{®}), verglichen. Dazu wurde die Mauszelllinie NFS-60 verwendet, die G-CSF-responsiv ist. Diese Zelllinie wurde in RPMI 1640-Medium (Firma Bachem, Heidelberg) kultiviert, das 1,5 g/l Natriumbicarbonat, 4,5 g/l Glucose, 10 mM Hepes und 1,0 mM Natriumpyruvat enthielt und mit 2 mM Glutamin, 10% FCS, 0,05 mM 2-Mercaptoethanol und 60 ng/ml G-CSF supplementiert worden war.

Für den Aktivitätstest wurden die Zellen zweimal mit Medium ohne G-CSF gewaschen, in einer Konzentration von 2 x 10⁴ Zellen pro Napf in 96-Napf-Platten ausgesät und für drei Tage bei 37°C und 4,5% CO₂ mit verschiedenen Konzentrationen des aufgereinigten G-CSF und des Standards inkubiert. Danach wurden sie mit XTT-Reagenz angefärbt und die Absorption bei 450 nm in einem Mikrotiterplattenlesegerät gemessen. Es zeigte sich, dass die Zellen, die mit dem erfindungsgemäß aufgereinigten G-CSF behandelt worden waren, genauso gut wuchsen wie die Zellen, die mit dem Standard behandelt worden waren, weshalb von einer gleichen biologischen Aktivität der beiden G-CSF-Proben ausgegangen werden kann.

Auch in den gelelektrophoretischen Analysen (SDS-PAGE, Western Blot, isoelektrische Fokussierung) verhielt sich das nach der chromatographischen Reinigung erhaltene Met-G-CSF wie das als Standard verwendete Neupogen^{®}.

Zur Bestimmung der Faltungsausbeute kann nach der Gewinnung des G-CSF eine rpHPLC durchgeführt werden, bei der das Protein denaturiert wird. Aufgrund der Disulfidbrücken, die erhalten bleiben, besitzen unterschiedlich Disulfid-überbrückte Spezies häufig unterschiedliche hydrophobe Oberflächen und können damit in der rpHPLC getrennt werden. Deshalb wird mit dieser Methode nur der Nachweis der richtigen Disulfidverbrückung erbracht. Allerdings ist diese ein entscheidendes und für viele kleine und entsprechend verbrückte Proteine bereits ein ausreichendes Kriterium für die richtige Faltung. Als weitere Analysemethode kann auch eine Size Exclusion (SE)-HPLC durchgeführt werden.

Geeignete Materialien und Protokolle zur Durchführung der rpHPLC oder der SE-HPLC kann der Fachmann den Produktinformationen von Anbietern wie Vydac (http://www.vydac.com) oder TOSOH Bioscience (http://www.tosohbiosep.de) entnehmen. Die Bestimmung der Ausbeute an Met-G-CSF ist auch beschrieben in Herman et al. (1996) Pharm. Biotechnol. 9: 303-328). Der genaue Anteil an Met-G-CSF wird dabei durch Integration der Peakfläche und Umrechnung anhand des Extinktionskoeffizienten ermittelt.

Nach der Aufreinigung kann das G-CSF hinsichtlich seiner Menge und seiner Aktivität analysiert werden. Eine qualitative Analyse kann über eine SDS-PAGE-Analyse mit anschließender Coomassie-Brilliant-Blue-Färbung oder eine rpHPLC erfolgen. Als Standard für die Analysen kann ein kommerziell erhältliches G-CSF-Präparat verwendet werden. Zusätzlich kann eine Peptide Map bzw. eine Massenspektroskopie durchgeführt werden. Die Aktivität des aufgereinigten G-CSF kann mit verschiedenen biologischen Testverfahren bestimmt werden, wie sie beispielsweise in Shirafuji et al. (1989) Exp. Hematol. 17(2): 116-119; Oh-Eda et al. (1990) J. Biol. Chem. 265(20): 11432-11435; Stute et al. (1992) Blood 79(11): 2849-2854 und Oshima et al. (2000) Biochem. Biophys. Res. Commun. 267(3): 924-927 beschrieben sind.

Sämtliche Chromatographien werden im Übrigen nach den Empfehlungen und Protokollen der Anbieter der Matrices bzw. der Säulen durchgeführt (z.B. bezüglich der Fließgeschwindigkeit, der zum Waschen bzw. für die Elution eingesetzten Säulenvolumen, der Durchmesser und Betthöhen der Säulen etc.).

## Patentansprüche

1. Verfahren zur Reinigung von rekombinantem Granulozyten-Koloniestimulierendem Faktor (G-CSF), umfassend zwei Kationenaustauschchromatographien, die jeweils vor und nach der hydrophoben Interaktionschromatographie durchgeführt werden, wobei dei Chromatographieschritte unmittlebar aufeindar folgen.

2. Verfahren nach Anspruch 1, wobei es sich bei dem zu reinigenden G-CSF um humanes Met-G-CSF, hergestellt in E. coli-Zellen handelt.

3. Verfahren nach Anspruch 1 oder 2, wobei als Ausgangsmaterial für die chromatographische Reinigung renaturiertes G-CSF eingesetzt wird.

4. Verfahren nach Anspruch 3, wobei der Faltungsansatz vor dem ersten Chromatographieschritt geklärt wird.

5. Verfahren nach einem der vorangehenden Ansprüche, umfassend weiterhin eine Tangentialflussfiltration im Anschluss an die letzte Kationenaustauschchromatographie.

6. Verfahren nach einem der vorangehenden Ansprüche, wobei keine Anionenaustauschchromatographie durchgeführt wird.

7. Verfahren nach einem der vorangehenden Ansprüche, wobei keine Gelfiltration durchgeführt wird.

8. Verfahren nach einem der vorangehenden Ansprüche, wobei keine HPLC durchgeführt wird.

9. Verfahren nach einem der vorangehenden Ansprüche, wobei keine Affinitätschromatographie durchgeführt wird.

10. Verfahren nach einem der vorangehenden Ansprüche, wobei keine Hydroxyapatitchromatographie durchgeführt wird.

11. Verfahren nach einem der vorangehenden Ansprüche, wobei für die Kationenaustauschchromatographie eine Sulfopropylmatrix verwendet wird.

12. Verfahren nach einem der vorangehenden Ansprüche, wobei für die hydrophobe Interaktionschromatographie Phenylgruppen als hydrophobe Liganden verwendet werden.

## Claims

1. Method for the purification of recombinant granulocyte-colony stimulating factor (G-CSF), comprising two cation exchange chromatographies which are, respectively, performed prior and after the hydrophobic interaction chromatography wherein the chromatographic steps are immediately consecutive.

2. Method according to claim 1, wherein the G-CSF to be purified is a human Met-G-CSF produced in in E. coli cells.

3. Method according to claim 1 or 2, wherein renatured G-CSF is used as starting material for the chromatographic purification.

4. Method according to claim 3, wherein the folding batch is clarified prior to the first chromatography step.

5. Method according to one of the preceding claims, further comprising a tangential flow filtration following the last cation exchange chromatography.

6. Method according to one of the preceding claims, wherein an anion exchange chromatography is not performed.

7. Method according to one of the preceding claims, wherein a gel filtration is not performed.

8. Method according to one of the preceding claims, wherein an HPLC is not performed.

9. Method according to one of the preceding claims, wherein an affinity chromatography is not performed.

10. Method according to one of the preceding claims, wherein a hydroxyapatite chromatography is not performed.

11. Method according to one of the preceding claims, wherein a sulfopropyl matrix is used for the cation exchange chromatography.

12. Method according to one of the preceding claims, wherein phenyl groups are used as hydrophobic ligands for the hydrophobic interaction chromatography.

## Revendications

1. Procédé de purification d'un facteur de stimulation de colonies de granulocytes recombinant (G-CSF), qui comprend deux chromatographies d'échange de cations réalisées respectivement avant et après la chromatographie par interaction hydrophobe dans lequel les étapes chromatographiques se suivent immédiatement.

2. Procédé selon la revendication 1, dans lequel il s'agit, pour le G-CSF à purifier, d'un Met-G-CSF humain, produit dans des cellules E. coli.

3. Procédé selon la revendication 1 ou 2, dans lequel on utilise comme matière de départ pour la purification chromatographique du G-CSF renaturé.

4. Procédé selon la revendication 3, dans lequel la préparation de repliement est clarifiée avant la première étape de chromatographie.

5. Procédé selon l'une des revendications précédentes, comprenant par ailleurs une étape de filtration à flux tangentiel à la suite de la dernière chromatographie d'échange de cations.

6. Procédé selon l'une des revendications précédentes, dans lequel on n'effectue pas de chromatographie d'échange d'anions.

7. Procédé selon l'une des revendications précédentes, dans lequel on n'effectue pas de filtration sur gel.

8. Procédé selon l'une des revendications précédentes, dans lequel on ne procède pas à une HPCL.

9. Procédé selon l'une des revendications précédentes, dans lequel on n'effectue pas de chromatographie d'affinité.

10. Procédé selon l'une des revendications précédentes, dans lequel on n'effectue pas de chromatographie d'hydroxyapatite.

11. Procédé selon l'une des revendications précédentes, dans lequel on se sert, pour la chromatographie d'échange de cations, d'une matrice de sulfopropyle.

12. Procédé selon l'une des revendications précédentes, dans lequel on utilise pour la chromatographie par interaction hydrophobe des groupes phényles comme ligands hydrophobes.
